# EUROPEAN PATENT APPLICATION

(11) **EP 3 808 172 A1**
(43) Date of publication of application: **21.04.2021**
(21) Application number: 20460007.6
(22) Date of filing: 29.01.2020
(51) Int. Cl.: A01H 4/00, A01H 6/14

(54) **METHOD OF OBTAINING SPROUTS BY WAY OF SOMATIC EMBRYOGENESIS FROM FRAGMENTS OF LEAF BLADES WITH ECHINACEA PURPUREA L. (MOENCH)**

(30) Priority: 14.10.2019 PL 43145619
(71) Applicant: University of Science and Technology, 85-796 Bydgoszcz (PL)
(72) Inventor: Lema-Ruminska, Justyna, 85-064 Bydgoszcz (PL)

(57) **Abstract**

The idea of the invention is obtaining *Echinacea purpurea* sprouts from fragments of leaf blades by way of somatic embryogenesis in *in vitro* cultures destined for further greenhouse cultivation, garden growing, production of secondary metabolites or creative breeding of new species in which plant material in the form of sterile fragments of leaf blades is placed on MS medium (1962) modified by increasing by 1/2 of iron chelate and calcium chloride and adding to the medium of growth regulators in the form of BAP cytokinin (6-benzylaminopurine) in the concentration of 0.5 - 1 mg/ L and auxin: IAA (3-indolylacetic acid) in the concentration of 0.5 - 1 mg/ L.

## Description

The idea of the invention is obtaining of Echinacea purpurea's sprouts from fragments of leaf blades by way of somatic embryogenesis in *in vitro* cultures destined for further greenhouse cultivation, garden production, production of secondary metabolites or creative breeding of new species.

*Echinacea purpurea* is both an ornamental and medical plant. It may be reproduced in a traditional way in *in vivo* conditions, by seeds, root division or cutting, however plants reproduced from seeds often do not form flowers in the second year of cultivation (Harbage 2001) and the remaining methods applied in *in vivo* conditions are not very efficient.

Due to the above reason, for production purposes it is necessary to elaborate an efficient method of multiplication based on *in vitro* technique. The most efficient method includes somatic embryogenesis, where potentially every live explant's somatic cell may regenerate somatic embryo from which a new plant (sprout) will develop.

We know methods of micropropagation of *Echinacea purpurea* from, e.g. patent application CN103858772A, known as *Tissue culture method of Echinacea purpurea (L.) Moench 'Double Decker',* however the kind of explant in the form of a petiole stick as well as acquired regenerated structures in the form of adventitious sprouts and addition to MS medium of NAA auxin (a-naphthylactic acid) besides BA cytokinin (6-benzyladenine) as provided in this solution differs from the idea of the present patent application for this invention.

From the application CN105052745A, we know the method of promoting growth of regeneration buds with *Echinacea purpurea* ("Method for promoting growth of *Echinacea purpurea* regeneration buds") that concerns promoting growth of adventitious buds depending on their length by adding to MS medium of DA-6 growth regulator (diethylaminoethanol hexanoate), which differs significantly from the aim of the solution applied in the present patent application and which concerns regeneration of buds by way of somatic embryogenesis from fragments of leaf blades. Also the composition of growth regulators is significantly different from the solution found in the present invention application.

We also know the process for micropropagation of *Echinacea purpurea* L. Moench in *in vitro,* where the medium and adding to the medium of MS steroid glycoside (Melongoside) acquired from *Solanum melongena* seeds or steroid glycoside acquired from *Solanum melongena* roots make it considerably different from the present suggested solution.

Moreover, we also know the method of acquiring adventitious roots of *Echinacea purpurea* in bioreactor culture ("*Method of bioreactor culture of Echinacea purpurea adventitious roots*") from the application no WO08100016A1. However, this method is aimed at propagating of adventitious roots in a bioreactor and producing active elements, which differs it significantly from the suggested solution.

The main idea behind this invention is the way of obtaining *Echinacea purpurea* sprouts from sterile fragments of leaf blades by way of somatic embryogenesis in *in vitro* cultures aimed for rooting, further greenhouse cultivation, garden cultivation or production or creative breeding of new variations of the species, where plant material in the form of sterile fragments of leaf blades is placed on MS medium that was modified by increasing by 1/4 of iron chelate and calcium chloride (1962). It is of key importance to add to the medium of growth regulators in the form of BAP cytokinin (6-benzylaminopurine) in the concentration of 0.5 - 1 mg/ L and auxin: IAA (3-indolylacetic acid) in the concentration of 0.5 - 1 mg/ L.

Explants in the form of rectangles with sides of 0.5-1 cm are placed on a medium in a horizontal way, abaxially. For the first 2 weeks of *in vitro* culture it is necessary to provide darkness, then the cultures grow in a growth room with relative air humidity of 60-80% in daylight conditions created by TLD 54/36W fluorescent lamps, with quantum radiation intensity of 30-50 µM×m-2×s-1, photoperiod of 16/8 (day/night), in the temperature of 24±2 degrees C for 8 - 10 weeks. Agar in the amount of 6-12 g/L and sucrose in the amount of 20-40 g/L with pH determined before the medium sterilization at 5.5-6.2 are added to the medium. The sprouts after the conversion of somatic embryos appear after 10 -12 weeks since the culture was initiated. In the following stage, the sprouts may be further propagated till obtaining of desirable amount or rooted and acclimatised to greenhouse conditions.

The advantage of this invention's solution is obtaining a large amount of new *Echinacea purpurea* sprouts after conversion of somatic embryos from fragments of leaf blades in *in vitro* culture conditions within only 10-12 weeks. The suggested solution allows to obtain sprouts ready for further propagation, rooting, acclimatisation and greenhouse cultivation, garden growing, garden production, production of secondary metabolites or creative breeding of new variations of species.

This method according to the invention is presented in a more detailed way by examples of implementing the invention.

### Example 1

Way of obtaining *Echinacea purpurea* sprouts from fragments of leaf blades by way of somatic embryogenesis in *in vitro* cultures according to the invention takes place within a few stages:
Stage I.
   Sterile leaf blades are taken from a plant grown in *in vitro* culture with the use of sterile tools in a laminar flow cabinet, which are then cut into rectangles (explants) with sides of 0.5 cm x 0.5 cm.
Stage II.
   The explants are then placed on sterile, modified MS medium (1962) with the addition of agar in the amount of 8 g/L and sucrose in the amount of 20 g/L; pH 5.6; adding growth regulators: BAP cytokinin (6-benzylaminopurine) in the condensation of 0.5 mg/L and auxin IAA (3-indolylactic acid) in the concentration of 0.5 mg/ L.
Stage III.
   After inoculation of the explants on mediums, *in vitro* cultures are placed in the growth room in the temperature of 24 degrees C ± 2 degrees C, with relative air humidity of 60% in darkness for 14 days.
Stage IV.
   The in vitro cultures are then placed on shelves in the growth room in the temperature of 24 degrees C ± 2 degrees C, with relative air humidity of 60% and quantum radiation intensity of 30 µM×m-2×s-1 emitted by daylight fluorescent lamps for 16 hours a day.
Stage V.
   After 3 weeks of the cultures on explants, somatic embryos appear that develop over the following weeks of the culture's growth. Sprouts after the conversion of somatic embryos are obtained after 10 weeks since the iniatiation of the culture.
Stage VI.
   In the following stage, the sprouts are destined for: propagation, rooting, acclimatisation and greenhouse cultivation, garden growing, garden production, production of secondary metabolites or creative breeding of new variations of the species.

### Example 2

Way of obtaining *Echinacea purpurea* sprouts from fragments of leaf blades by way of somatic embryogenesis in *in vitro* cultures according to the invention takes place within a few stages:
Stage I.
   Previously sterilized and rinsed (sterilized with the use of 0.4% sodium hypochlorite for 10 minutes; rinsed 3 times for 10 minutes in sterile distilled water) leaf blades taken from a plant cultivated in *in vivo* conditions, are cut with the use of sterile tools in a laminar flow cabinet, into rectangles of 1 cm x 1m cm sides.
Stage II.
   The explants are then placed on sterile, modified MS medium (1962) with the: addition of agar in the amount of 10 g/L and sucrose in the amount of 40 g/L; pH 6.0; adding growth regulators: BAP cytokinin (6-benzylaminopurine) in the concentration of 1 mg/L and auxin IAA (3-indolylactic acid) in the condensation of 1 mg/ L.
Stage III.
   After inoculation of the explants on mediums, *in vitro* cultures are placed in the growth room in the temperature of 24 degrees C ± 2 degrees C, with relative air humidity of 60% in darkness for 14 days.
Stage IV.
   The *in vitro* cultures are then placed on shelves in the growth room in the temperature of 24 degrees C ± 2 degrees C, with relative air humidity of 60% and quantum radiation intensity of 50 µM×m-2×s-1 emitted by daylight fluorescent lamps for 16 hours a day.
Stage V.
   After 3 weeks of the cultures on explants, somatic embryos appear that develop over the following weeks of the culture's growth. Sprouts after the conversion of somatic embryos are obtained after 12 weeks since the iniatiation of the culture.
Stage VI.
   In the following stage, the sprouts are destined for: propagation, rooting, acclimatisation and greenhouse cultivation, garden growing, garden production, production of secondary metabolites or creative breeding of new variations of the species.

## Claims

1. Method of obtaining *Echinacea purpurea* sprouts from fragments of leaf blades by way of somatic embryogenesis in *in vitro* cultures is **characterized by** the fact that the plant material in the form of sterile leaf blades is cut with a scalpel in a laminar flow cabinet into rectangles of 0.5 x 1 cm in size and placed in a horizontal way (abaxially) on modified MS medium with the addition of agar in the amount of 6-12 g/L and sucrose in the amount of 20-40 g/L of pH 5.5-6.2, then the *in vitro* cultures are kept in darkness for 14 days in the temperature of 24 degrees C ± 2 degrees C and relative humidity of 60-80% and finally they are placed in a growth room with quantum radiation intensity of 30-50 µM×m-2×s-1 emitted by daylight fluorescent lamps for 16 hours a day for 8 - 10 weeks.

2. The method according to the invention in item 1 is **characterized by** the fact that MS medium (1962) is modified by increasing by 1/2 of iron chelate and calcium chloride and adding to the medium of growth regulators in the form of BAP cytokinin (6-benzylaminopurine) in the concentration of 0.5 - 1 mg/ L and auxin: IAA (3-indolylacetic acid) in the concentration of 0.5 - 1 mg/ L.
